# EUROPÄISCHE PATENTANMELDUNG

(11) **EP 4 014 948 A1**
(43) Veröffentlichungstag der Anmeldung: **22.06.2022**
(21) Anmeldenummer: 20215304.5
(22) Anmeldetag: 18.12.2020
(51) Int. Cl.: A61K 6/60, A61K 6/20

(54) **ZUSAMMENSETZUNG ZUR REMINERALISATION VON ZÄHNEN**

(71) Anmelder: Ivoclar Vivadent AG, 9494 Schaan (LI)
(72) Erfinder: Lendenmann, Urs, 9472 Grabs (CH); Bolis-Truog, Carlo, 7206 Igis (CH); Alge, Michelle, 6890 Lustenau (AT)
(74) Vertreter: Uexküll & Stolberg

(57) **Zusammenfassung**

Zusammensetzung zur Anwendung in einem Verfahren zur therapeutischen Behandlung von Zähnen, die mindestens eine fluoridhaltige Komponente, mindestens eine Aminosäure und einen flüssigen Träger enthält. Die Zusammensetzung hat vorzugsweise einen pH von 4 bis 7 und enthält vorzugsweise keine abrasiven oder polymeren Bestandteile.

## Beschreibung

Die vorliegende Erfindung betrifft eine Zusammensetzung zur Remineralisation von Zähnen. Die Zusammensetzung enthält eine oder mehrere Aminosäuren und eine Fluoridquelle. Sie wird auf die Zähne aufgetragen und bewirkt eine schnelle Remineralisation der Zahnhartsubstanz.

### Stand der Technik

Karies ist eine Erkrankung von Zahnhartgewebe (Schmelz und Dentin). Sie entsteht, wenn Säure dem anorganischen Anteil der Zahnhartsubstanz, welcher vorwiegend aus Hydroxylapatit besteht, Mineralien entzieht. Dies reduziert die Dichte des Minerals und erhöht die Durchlässigkeit der Zahnstruktur für Flüssigkeiten und Ionen. Die Säure kann direkt durch Nahrung, vor allem durch saure Getränke, auf die Zähne gelangen. Von größerer Bedeutung sind aber Kohlenhydrate, vor allem Saccharose, welche von Biofilmen auf den Zähnen zu organischen Säuren, vor allem Milchsäure, fermentiert werden. Dabei kann der pH-Wert im Biofilm auf den Zähnen in wenigen Minuten auf ca. 4 bis 5 absinken (Lingstrom et al., 1993, J Dent Res, 72:865-870). Wenn eine Untersättigung in Bezug auf die Löslichkeit von Hydroxylapatit im Speichel vorherrscht, dann bilden sich als Kariesvorstufe (Initialkaries) zuerst Entkalkungen. Diese sind makroskopisch als weiße Flecken (white spot) erkennbar (Arends & Christoffersen, 1986, J Dent Res, 65:2-11). Solange die initiale Kariesläsion nicht so weit fortgeschritten ist, dass die Oberfläche einbricht und ein Loch entsteht, kann sie remineralisiert werden.

Obwohl die Möglichkeit der Remineralisation von Schmelzprüfkörpern im Labor schon vor 1970 nachgewiesen wurde (Johansson, 1965, J Dent Res, 44:64-70; Feagin et al., 1969, Arch Oral Biol, 14:1407-1417), gibt es bis heute keine Produkte, die klinisch nachweisbar und voraussagbar besser remineralisieren als Fluoride. Tatsächlich wurde aber schon vor vielen Jahren beschrieben, dass *in vivo* die Remineralisation wesentlich langsamer verläuft als im Labor (Gelhard & Arends, 1984, J Biol Buccale, 12:49-57).

In der Prävention von Karies haben sich bis jetzt vor allem gute Mundhygiene und die topische Applikation von Fluorid auf die Zähne bewährt. Fluorid reduziert die Löslichkeit von Hydroxylapatit. Die regelmäßige Fluoridapplikation auf Zähne führt auch zu Remineralisation (Gao et al. 2016, BMC Oral Health, 16:12). Dies benötigt jedoch sehr viel Zeit, und der Erfolg ist schlecht vorhersagbar, so dass die Fluoridapplikation, z.B. in Form von Zahnpasten oder Mundspüllösungen, nur eine mäßige Wirkung hat.

Zahnpasten enthalten zudem Abrasivstoffe und Verdickungsmittel. Die Abrasivstoffe dienen zum Entfernen von Zahnbelägen, während die Verdickungsmittel zur Erhöhung der Viskosität beitragen, um somit eine Paste zu erhalten. Die regelmäßige Anwendung von abrasiven Zahnpasten kann, vor allem bei schlechter Putztechnik, zu erhöhtem Schmelzabtrag und Zahnfleischverletzungen führen. Die fluoridierende Wirkung von Zahnpasten ist nur gering. Zudem ist die Remineralisationswirkung von Zahnpasten und Mundspüllösungen stark von der Kooperation und der Disziplin des Anwenders abhängig, da diese Produkte wiederholt täglich angewendet werden müssen.

Dies alles wirkt sich nachteilig auf den Erfolg der Remineralisation aus. Es besteht daher Bedarf an einfachen, schnellen Verfahren zur Remineralisation von Zähnen, die in einem Schritt und nach nur einmaliger Behandlung in kurzer Zeit eine substanzielle Remineralisation bewirken.

Die EP 3 513 777 A1 offenbart ein Verfahren zur Remineralisation von Zähnen, bei dem zuerst eine Lösung einer Fluoridkomponente und anschließend ein Sol oder ein Kolloid einer nano-Calciumkomponente auf die Zähne aufgetragen wird. Wesentlich ist, dass die Fluoridkomponente vor der Calciumkomponente auf den Zahn aufgebracht wird.

Die DE 10 2010 003 280 A1 offenbart Mund- und Zahnpflegemittel, die mindestens ein Oligo- oder Polypeptid pflanzlicher Herkunft enthalten, bei dem das Molverhältnis von basischen Aminosäuren (Arginin, Histidin, Lysin) zu sauren und semi-sauren Aminosäuren (Asparginsäure, Glutaminsäure, Tyrosin, Cystein) größer als 1 ist. Die Peptide sollen das Wachstum von Keimen begünstigen, die für die Mundgesundheit vorteilhaft sind. Außerdem sollen sie die Remineralisation von Zähnen durch calciumhaltige Substanzen verbessern.

Die DE 38 16 237 A1 offenbart Feinfüllmaterialen für zahnmedizinische Zwecke, die Hydroxylapatit und vorzugsweise auch ein die Calcifikation förderndes Protein enthalten. Das Protein ist vorzugsweise aus Phosvitin, Casein und histidinreichen Proteinen ausgewählt. Der Feinfüller soll sich zur Ausbesserung von Vertiefungen, Rissen und Oberflächenläsionen im Zahnschmelz eignen.

### Kurzbeschreibung der Erfindung

Der Erfindung liegt die Aufgabe zu Grunde, Zusammensetzungen zur Verfügung zu stellen, die sich zur Remineralisation von Zähnen, zur Behandlung initialer Kariesläsionen, zum Kariesschutz und zur Verhinderung und Behandlung von Zahnerosion eignen. Die Zusammensetzungen sollen die einfache und schnelle Remineralisation von Zähnen durch eine einmalige Behandlung ermöglichen.

Diese Aufgabe wird erfindungsgemäß durch Zusammensetzungen gelöst, die neben einem flüssigen Träger und mindestens einer fluoridhaltigen Komponente mindestens eine Aminosäure enthalten. Zur Remineralisation von Zähnen wird die Zusammensetzung auf die zu behandelnden Zahnareale aufgetragen. Es wurde überraschenderweise gefunden, dass die erfindungsgemäßen Zusammensetzungen eine deutlich bessere Remineralisation bewirken als bekannte fluoridhaltige Präparate. Damit ermöglichen die erfindungsgemäßen Zusammensetzungen nicht nur einen effizienten Kariesschutz, d.h. sie verhindern nicht nur, dass Läsionen entstehen oder sich vergrößern, sondern erlauben auch eine wirksame Behandlung initialer Kariesläsionen und die Verhinderung und Behandlung der Zahnerosion.

### Detaillierte Beschreibung der Erfindung

Die Erfindung betrifft eine Zusammensetzung und ein Verfahren zur Remineralisation von Zähnen, insbesondere zur Behandlung initialer Kariesläsionen, beispielsweise von Fissuren, Glattflächen, Interdentalflächen und Zahnhälsen, zum Kariesschutz an ausgewählten Zahnflächen, und zur Verhinderung und Behandlung der Zahnerosionen. Unter Zahnerosion wird der progressive Verlust von mineralisiertem Zahngewebe durch chemische Prozesse verstanden, die nicht durch Mikroorganismen verursacht werden.

Die erfindungsgemäßen Zusammensetzungen zeichnen sich dadurch aus, dass sie eine Fluoridkomponente in Kombination mit einer Aminosäure enthalten. Es wurde überraschend gefunden, dass die erfindungsgemäßen Zusammensetzungen schon nach einer einmaligen, kurzzeitigen Behandlung die Härte von Zähnen deutlich erhöhen.

Bevorzugte Fluoridkomponenten sind Natriumfluorid, Kaliumfluorid, Rubidiumfluorid, Cäsiumfluorid, Kaliumbifluorid (KHF₂), Silber-I-fluorid (AgF), Zinn-II-fluorid (SnF₂), Ammoniumfluorid, Ammoniumbifluorid, Salze der Fluorphosphorsäure, wie Natriummonofluorphosphat, Kaliummonofluorphosphat und Ammoniumhexafluorphosphat, Tetra-n-butylammoniumdihydrogentrifluorid (TBAF-3), Olaflur (INN), Dectaflur (INN) oder eine Mischung davon. Besonders bevorzugte Fluoridverbindungen sind Ammoniumfluorid, Ammoniumbifluorid, Natriumfluorid, Kaliumfluorid, Tetra-n-butylammoniumdihydrogentrifluorid und Mischungen davon. Ganz besonders bevorzugt sind Ammoniumbifluorid, Tetra-n-butylammoniumdihydrogentrifluorid, Olaflur (INN), Dectaflur (INN) und insbesondere Ammoniumfluorid.

Bevorzugt sind Zusammensetzungen, die mehr als 1500 ppm, bevorzugt mehr als 5000 ppm an Fluorid (bezogen auf das Fluoridanion) in gelöster Form enthalten. Die maximale Konzentration hängt von der Wahl des Lösungsmittels und des Fluorsalzes ab. Sie liegt vorzugsweise unter 20 Gew.-%, besonders bevorzugt unter 10 Gew.-%. Die erfindungsgemäßen Zusammensetzungen enthalten vorzugsweise 0,15 Gew.-% bis 20 Gew.-%, besonders bevorzugt 0,5 Gew.-% bis 10 Gew.-%, ganz besonders bevorzugt 1 bis 10 Gew.-% und am meisten bevorzugt 1 bis 5 Gew.-% Fluoridionen. Wenn nicht anders angegeben beziehen sich alle Prozentangaben hierin auf die Gesamtmasse der Zusammensetzung.

Als Aminosäuren können nicht natürlich vorkommende Aminosäuren oder vorzugsweise natürlich vorkommende, besonders bevorzugt proteinogene Aminosäuren eingesetzt werden. Erfindungsgemäß können basische, neutrale oder saure Aminosäuren verwendet werden. Bevorzugt sind Lysin, Histidin, Arginin, Glutamin, Asparagin, Tyrosin, Glycin, Serin, Cystein, Threonin, Alanin, Valin, Methionin, Leucin, Isoleucin, Prolin, Tryptophan, Phenylalanin, Glutaminsäure, Asparaginsäure, Hydroxyprolin und Mischungen davon. Besonders bevorzugt sind Glutamin, Asparagin, Tyrosin, Glycin, Serin, Cystein, Threonin, Alanin, Valin, Methionin, Leucin, Isoleucin, Prolin, Tryptophan, Phenylalanin, Glutaminsäure, Asparaginsäure, Hydroxyprolin und Mischungen davon. Ganz besonders bevorzugte Aminosäuren sind Histidin und insbesondere Phenylalanin und Lysin sowie Mischungen davon. Die Aminosäuren liegen in monomerer Form vor. Erfindungsgemäß besonders bevorzugt ist die Kombination von Histidin und Ammoniumfluorid.

Die Aminosäuren werden vorzugsweise in einer Gesamtmenge von 0,01 bis 0,5 mol/l, bevorzugt 0,025 bis 0,2 mol/l eingesetzt, bezogen auf das Gesamtvolumen der Zusammensetzung.

Die erfindungsgemäßen Zusammensetzungen enthalten außerdem einen flüssigen Träger. Als Träger sind Lösungsmittel und Lösungsmittelmischungen bevorzugt, welche die Fluorid-verbindung(en), Aminosäure(n) und ggf. vorhandene Hilfsstoffe, wie beispielsweise Stoffe zur Einstellung des pH-Werts, lösen können. Erfindungsgemäß sind solche Zusammensetzungen besonders bevorzugt, in der alle Komponenten in vollständig gelöster Form vorliegen.

Bevorzugte Lösungsmittel sind Wasser, mit Wasser mischbare organische Lösungsmittel und Mischungen davon. Bevorzugte organische Lösungsmittel sind Ethanol, Isopropanol, Aceton, Methanol und Propylenglycol. Besonders bevorzugt sind Mischungen von Wasser mit mindestens einem organischen Lösungsmittel. Ganz besonders bevorzugt sind Wasser, Ethanol, Isopropanol, Aceton und Mischungen davon, insbesondere Mischungen die Wasser und mindestens eines der genannten organischen Lösungsmittel enthalten, und ganz besonders bevorzugt wird ausschließlich Wasser verwendet.

Organische Lösungsmittel können die Löslichkeit von Fluoridsalzen und Aminosäuren verringern. Sie sind aber andererseits vorteilhaft, weil sie die Benetzung der Zahnsubstanz verbessern und/oder das Trocknen der Lösung nach der Applikation beschleunigen. Für Anwendungen, bei denen z.B. ein schnelles Trocknen erwünscht ist, sind daher organische Lösungsmittel und Lösungsmittelmischungen, die mindestens ein organisches Lösungsmittel enthalten, bevorzugt. Dabei wird vorzugsweise ein organisches Lösungsmittel oder ein Lösungsmittelgemisch verwendet, in dem die verwendeten Fluoridkomponente(n) und Aminosäure(n) in den gewünschten Mengen vollständig löslich sind. Erfindungsgemäß sind Lösungsmittelmischungen bevorzugt, maximal 25 Vol.-% organisches Lösungsmittel und vorzugsweise mehr als 75 Vol.-% Wasser enthalten, jeweils bezogen auf das Gesamtvolumen an Lösungsmitteln.

Die erfindungsgemäßen Zusammensetzungen haben vorzugsweise einen pH-Wert von 3 bis 8, besonders bevorzugt von 4 bis 7, ganz besonders bevorzugt 4 bis 6, und am meisten bevorzugt von 4,5 bis 5,5. Die Einstellung des pH-Werts kann ggf. durch Zusatz von Säure(n) oder Base(n) erfolgen. Die Säure(n) oder Base(n) können anorganisch oder organisch sein. Erfindungsgemäß bevorzugte Säuren sind Salzsäure, Phosphorsäure, Salpetersäure, Schwefelsäure, Milchsäure, Essigsäure, Ameisensäure, Zitronensäure und Mischungen davon. Erfindungsgemäß bevorzugte Basen sind Natriumhydroxid, Kaliumhydroxid, Ammoniumhydroxid, 2-Aminomethylpropanol, Tris-(hydroxymethyl)-aminomethan, Tetrahydroxypropylethylendiamin und Mischungen davon.

Erfindungsgemäß sind solche Zusammensetzungen besonders bevorzugt, die keine Calciumsalze und/oder keine Phosphate und insbesondere kein Calciumphosphat enthalten. Außerdem sind erfindungsgemäß solche Zusammensetzungen bevorzugt, die keine abrasiven und/oder polymeren Komponenten enthalten. Unter Phosphaten werden hier die Salze der Orthophosphorsäure verstanden. Die Salze der Fluorphosphorsäure, wie z.B. Natriummonofluorphosphat und Kaliummonofluorphosphat, fallen nicht darunter.

Insbesondere enthalten die erfindungsgemäßen Zusammensetzungen vorzugsweise keine Di- oder Monohydrogenphosphate wie Monocalciumphosphat Ca(H₂PO₄)₂, Dicalciumphosphat CaHPO₄, Tricalciumphosphat Ca₃(PO₄)₂, Octacalciumphosphat Ca₈H₂(PO₄)₆, kein amorphes Calciumphosphat (ACP), keine Di- oder Polyphosphate wie Dicalciumdiphosphate Ca₂P₂O₇ oder Calciumtriphosphat Ca₅(P₃O₁₀)₂, keine Hydroxy- oder Oxophosphate, wie Hydroxylapatit Ca₅(PO₄)₃(OH), Apatit Ca₁₀(PO₄)₆(OH, F, Cl, Br)₂, oder Tetracalciumphosphate Ca₄(PO₄)₂O, weder in wasserfreier Form noch in Form der Hydrate. Außerdem enthalten die erfindungsgemäßen Zusammensetzungen vorzugsweise kein Calciumcarbonat CaCO₃, Calciumchlorid CaCl₂, Calciumfluorid CaF₂, Calciumglycerophosphat, Calciumhydroxid Ca(OH)₂, Calciumsulfat CaSO₄, Calciumlactat oder Calciumgluconat, weder in wasserfreier Form noch in Form der Hydrate.

Die erfindungsgemäßen Zusammensetzungen zur Verwendung bei der Remineralisation von Zähnen enthalten vorzugsweise:
(a) 50 bis 99,5 Gew.-%, vorzugsweise 75 bis 99 Gew.-%, besonders bevorzugt 85 bis 98 Gew.-% und ganz besonders bevorzugt 86 bis 96 Gew.-% flüssigen Träger,
(b) 0,15 Gew.-% bis 20 Gew.-%, vorzugsweise 0,5 Gew.-% bis 10 Gew.-% und besonders bevorzugt 1 bis 5 Gew.-% (bezogen auf F⁻) mindestens einer fluoridhaltigen Komponente, jeweils bezogen auf das Gesamtgewicht der Zusammensetzung,
(c) 0,01 bis 0,5 mol/l, vorzugsweise 0,025 bis 0,2 mol/l und besonders bevorzugt 0,05 bis 0,1 mol/l mindestens einer Aminosäure, bezogen auf das Gesamtvolumen der Zusammensetzung und
(d) ggf. Mittel zur Einstellung des pH-Werts.

Neben den genannten Komponenten können die erfindungsgemäßen Zusammensetzungen vorteilhaft weitere Additive (e) enthalten. Bevorzugte Additive sind Butyldiglykol, Süßstoffe, Aromastoffe und Tenside. Tenside begünstigen die Auflösung der auf der Zahnoberfläche üblicherweise vorhandene Pellikel und/oder Beläge. Additive werden ggf. in einer Gesamtmenge von 0,01 bis 5 Gew.-%, vorzugsweise 0,1 bis 3 Gew.-% und besonders bevorzugt 0,5 bis 2 Gew.-% zugesetzt, bezogen auf das Gesamtgewicht der Zusammensetzung.

Erfindungsgemäß sind naturgemäß solche Zusammensetzungen besonders bevorzugt, bei denen die einzelnen Bestandteile aus den oben definierten bevorzugten und besonders bevorzugten Stoffen ausgewählt sind. Ganz besonders bevorzugt sind Zusammensetzungen, die ausschließlich aus den genannten Bestandteilen bestehen.

Die erfindungsgemäßen Zusammensetzungen enthalten vorzugsweise keine Verdickungsmittel, keine Feuchthaltemittel und keine Gelbildner, insbesondere kein Sorbitol und/oder Glycerin.

### Verfahren zur Remineralisation von Zähnen

Zur Remineralisation eines oder mehrerer Zähne wird die erfindungsgemäße Zusammensetzung auf die zu behandelnde Zahnoberfläche aufgebracht. Das erfindungsgemäße Verfahren umfasst vorzugsweise die folgenden Schritte:
(i) Optionale Vorbehandlung des Zahns oder der Zähne,
(ii) optionales Anätzen des Zahns oder der Zähne und
(iii) Applikation einer erfindungsgemäßen Zusammensetzung.

### Optionale Vorbehandlung des Zahns oder der Zähne

Vorzugsweise werden die zu behandelnden Zahnareale vor der eigentlichen Behandlung gereinigt. Dabei werden Zahnstein (Kalkulus) und andere Beläge auf den Zähnen entfernt. Besonders bevorzugt wird eine professionelle Zahnreinigung durchgeführt.

Da nach einer (professionellen) Zahnreinigung mit einer abschließenden Politur ein dünner Film von Debris, die sogenannte Schmierschicht, auf dem Zahn verbleiben kann, ist es bevorzugt, die zu behandelnden Zahnareale nach der Reinigung oberflächlich anzuätzen, damit die erfindungsgemäße Zusammensetzung tief in den Zahnschmelz eindringen kann. Dazu wird ein Ätzmittel punktuell auf die betreffenden Bereiche, insbesondere die demineralisierten, als weiße Flecken sichtbaren Initialläsionen, aufgebracht und für kurze Zeit darauf belassen. Danach wird das Ätzmittel abgespült. Die Einwirkzeit der Säure ist nicht sehr kritisch. Sie sollte lang genug sein, um die Schmierschicht zu lösen, aber nicht so lang, dass der Zahnschmelz unnötig geschwächt wird. Erfindungsgemäß sind Zeiten zwischen 1 s und 1000 Sekunden, bevorzugt 5 s bis 120 s, besonders bevorzugt 5 s bis 60 s bevorzugt.

Als Säuren zum Ätzen kommen insbesondere wässrige Lösungen von Phosphorsäure, Salzsäure, Salpetersäure, Schwefelsäure, Milchsäure, Essigsäure, Ameisensäure, Zitronensäure, Ethylendiamintetraessigsäure in Frage. Besonders bevorzugt ist Phosphorsäure, vorzugsweise 37 %-ige Phosphorsäure.

Damit diese Säurelösungen punktuell genau appliziert werden können, enthalten sie vorzugsweise einen Verdicker. Geeignete Ätzmittel sind an sich aus der dentalen Adhäsivtechnik bekannt und gut beschrieben. Bevorzugt ist 37%-iges Phosphorsäure-Ätzgel, wie z.B. das unter der Bezeichnung Total Etch von der Ivoclar Vivadent AG kommerziell erhältliche Gel.

Das Ätzmittel wird nach der gewünschten Einwirkzeit mit Wasser abgespült und der Zahn danach getrocknet, vorzugsweise mit einem Luftstrom, aber auch andere Methoden sind geeignet.

### Applikation der erfindungsgemäßen Zusammensetzung

Nach der Reinigung und/oder der Säurebehandlung der Zahnoberfläche wird die erfindungsgemäße Zusammensetzung auf den zu behandelnden Zahn oder vorzugsweise auf die gesamte natürliche Bezahnung aufgetragen, z.B. mit einem kleinen Pinsel, Schwamm oder einer Spritze mit stumpfer Kanüle. Für die Behandlung der gesamten Bezahnung werden in der Regel etwa 0,5 ml oder weniger, vorzugsweise 0,1 bis 0,3 ml, der erfindungsgemäßen Zusammensetzung benötigt. Alternativ kann die Zusammensetzung gezielt auf die zu behandelnde Stellen aufgetragen werden, besonders dann, wenn zuvor keine professionelle Zahnreinigung erfolgt ist. Die Einwirkzeit nach dem Auftragen der Zusammensetzung beträgt vorzugweise jeweils weniger als 5 Minuten, besonders bevorzugt 0,5 bis 2 Minuten und ganz besonders bevorzugt 0,5 bis 1 Minute. Nach Ablauf der Einwirkzeit der erfindungsgemäßen Zusammensetzung können die Zähne optional getrocknet werden, z.B. mit einem Luftbläser. Allfällige Überschüsse müssen nicht entfernt werden. Eine unmittelbar anschließende zweite Applikation der Zusammensetzung ist möglich, aber in der Regel nicht erforderlich, um die gewünschte Wirkung zu erzielen. Eine wiederholte, d.h. zweite oder dritte, Applikation ist insbesondere dann vorteilhaft, wenn beim ersten Aufbringen nicht alle zu behandelnden Stellen von der Zusammensetzung erreicht wurden.

Es wurde überraschend gefunden, dass die erfindungsgemäßen Zusammensetzungen schon nach einer einmaligen Applikation eine hohe Wirkung erzielen. Besonders überraschend war, dass diese Wirkung bereits nach einer kurzen Einwirkungszeit erzielt wird. Diese beträgt, wie oben angegeben, gemäß einer besonders bevorzugten Ausführungsform nur 0,5 bis 2 Minuten, wobei eine Einwirkungszeit von 1 Minute ganz besonders bevorzugt ist. Dies ist im Hinblick auf eine Anwendung durch den Zahnarzt ein wesentlicher Vorteil, weil die Behandlung bereits nach kurzer Zeit abgeschlossen ist, was sowohl für den Patienten als auch für das behandelnde Personal eine erhebliche Erleichterung darstellt.

Die erfindungsgemäßen Zusammensetzungen eignen sich besonders zur therapeutischen Anwendung durch den Zahnarzt, insbesondere zur Remineralisation von Zähnen, zum Kariesschutz und ganz besonders zur therapeutischen Behandlung und Prävention initialer Kariesläsionen (Initialkaries), von weißen Flecken (white spots), von Zahnerosion, zur Härtung geschädigter Zähne und zur Wiederherstellung von Zahnhartgewebe. Die Zusammensetzungen bewirken nicht nur eine deutliche Zunahme der Härte der oberen Zahnschichten sondern auch einen anhaltenden Schutz der Zähne vor nachfolgenden Säureangriffen. Diese Wirkung wird allein durch die Anwendung der erfindungsgemäßen Zusammensetzung erreicht. Vorzugsweise werden daher keine weiteren Behandlungsschritte mit therapeutisch wirksamen Mitteln vorgenommen. Nicht therapeutische Maßnahmen, wie z.B. ein Spülen der Zähne nach der Anwendung, sind nicht ausgenommen.

Ein bevorzugter Gegenstand der Erfindung sind somit Mittel zur Anwendung in einem Verfahren zur therapeutischen Behandlung von Zähnen, d.h. zum Kariesschutz, zur Behandlung und Prävention initialer Kariesläsionen (Initialkaries), von weißen Flecken (white spots), von Zahnerosion, zur Härtung geschädigter Zähne und zur Wiederherstellung von Zahnhartgewebe, wobei die Zusammensetzung ein- bis dreimal, vorzugsweise einmal, auf den Zahn oder die Zähne aufgebracht und jeweils für einen Zeitraum von weniger als 5 Minuten, vorzugsweise 0,5 bis 2 Minuten, besonders bevorzugt 0,5 bis 1 Minute und ganz besonders bevorzugt 1 Minute einwirken gelassen wird.

Im Folgenden wird die Erfindung anhand von Figuren und Beispielen näher erläutert.

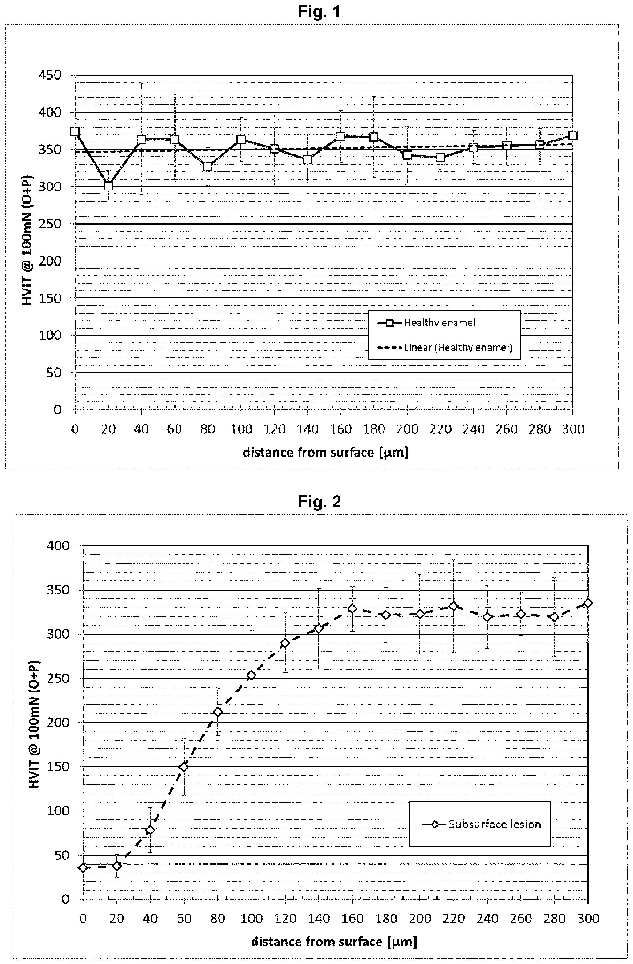

Fig. 1 zeigt das Härteprofil eines gesunden Rinderzahns. Die Härte des Zahn an der Oberfläche (distance = 0 µm) entspricht der Härte in tieferen Bereichen (distance = 300 µm).
Fig. 2 zeigt das Härteprofil eines Rinderzahns nach Lagerung in einer Demineralisationslösung. Hier ist die Vickershärte (HV_{IT}) an der Zahnoberfläche, die unmittelbar in Kontakt mit der Demineralisationslösung stand, deutlich verringert (von ca. 320 HV_{IT} auf ca. 40 HV_{IT}). Das Härteprofil zeigt, dass sich die Demineralisation bei den gewählten Bedingungen bis zu einer Tiefe von ca. 160 µm auswirkt, gemessen von der Zahnoberfläche. Ab einer Tiefe von ca. 160 µm weist der Zahn die natürliche Härte auf.

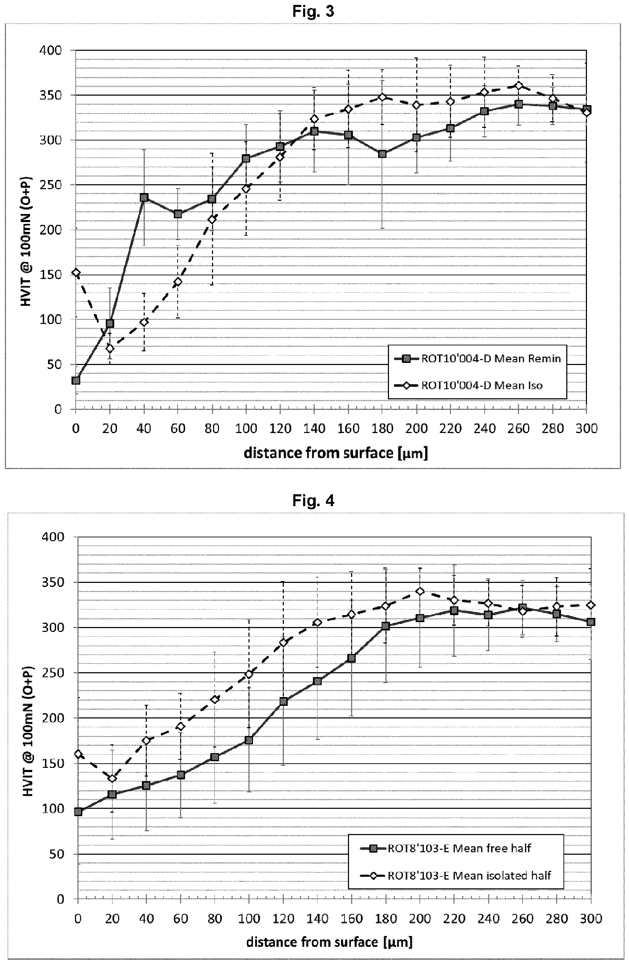

Fig. 3 zeigt die Härteprofile eines Rinderzahns, der mit einer nicht erfindungsgemäßen Ammoniumfluoridlösung (5% Ammoniumfluorid, ohne Aminosäurezusatz) behandelt wurde (Beispiel 1). Die gestrichelte Linie zeigt das Profil der unbehandelten Schmelzprobe und die durchgehende Linie das Härteprofil der Schmelzprobe, die mit der Fluoridlösung behandelt wurde. Die Härteprofile zeigen, dass die Ammoniumfluoridlösung zwar eine mäßige Remineralisation in einem engen oberflächennahen Bereich bewirkt, in tieferen Bereichen wird aber eine weitere Demineralisation beobachtet.
Fig. 4 zeigt die Härteprofile eines Kontrollversuchs mit einer unbehandelten Initialläsion (Negativkontrolle; Beispiel 2). Die Initialläsion wurde zu Hälfte mit Nagellack abgedeckt, und die Zähne wurden dann für weitere 7 Tage in der demineralisierenden Lösung gelagert. Durch den Nagellack wird ein weiterer Kontakt mit der Demineralisierungslösung verhindert. Die gestrichelte Linie zeigt das Profil der mit Nagellack isolierten Hälfte der Schmelzproben, die durchgehende Linie das Härteprofil der unbehandelten Schmelzproben. Es ist zu erkennen, dass die fortgesetzte Lagerung der Zähne in der Demineralisierungslösung zu einer weiteren Abnahme der Härte führt. Dies bedeutet, dass die Läsion größer wird und die Zähne weiter demineralisiert werden.

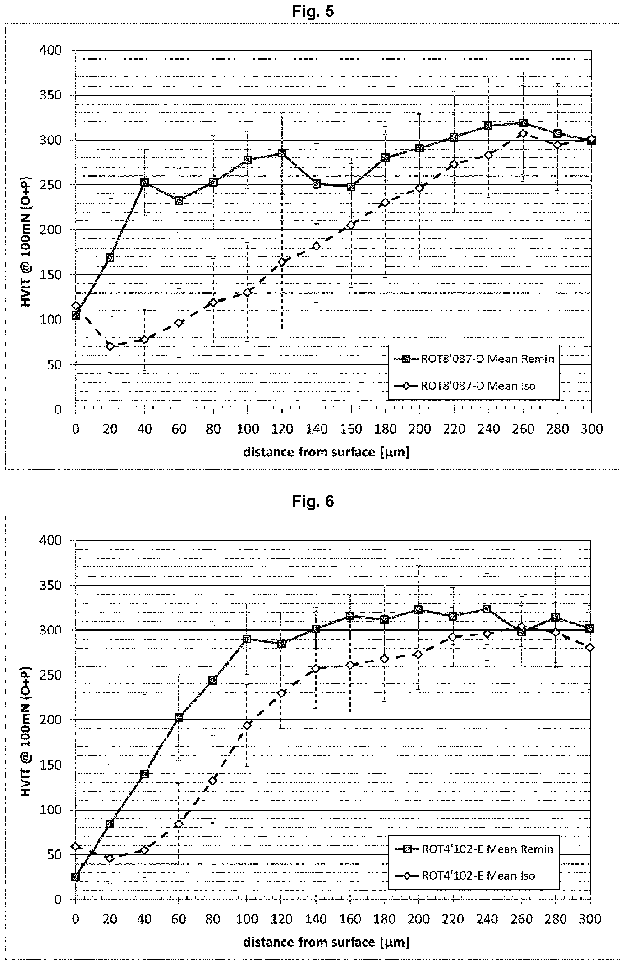

Fig. 5 zeigt die Härteprofile von Zähnen, die mit einer erfindungsgemäßen Zusammensetzung behandelt wurden (Beispiel 3; 0,1M Lysin und 5% Ammoniumfluorid; durchgezogene Kurve), im Vergleich zu einer unbehandelten Schmelzprobe (gestrichelte Kurve). Die Härteprofile zeigen, dass die Behandlung mit der erfindungsgemäßen Lösung eine deutliche Härtezunahme der Zähne bewirkt.
Fig. 6 zeigt ebenfalls die Härteprofile von Zähnen, die mit einer erfindungsgemäßen Zusammensetzung behandelt wurde (Beispiel 4; 0,1M Histidin und 5% Ammoniumfluorid; durchgezogene Kurve), im Vergleich zu einer unbehandelten Schmelzprobe (gestrichelte Kurve). Die Härteprofile zeigen, dass die Behandlung mit der erfindungsgemäßen Lösung eine Härtezunahme in weiten Bereichen der Läsion bewirkt.

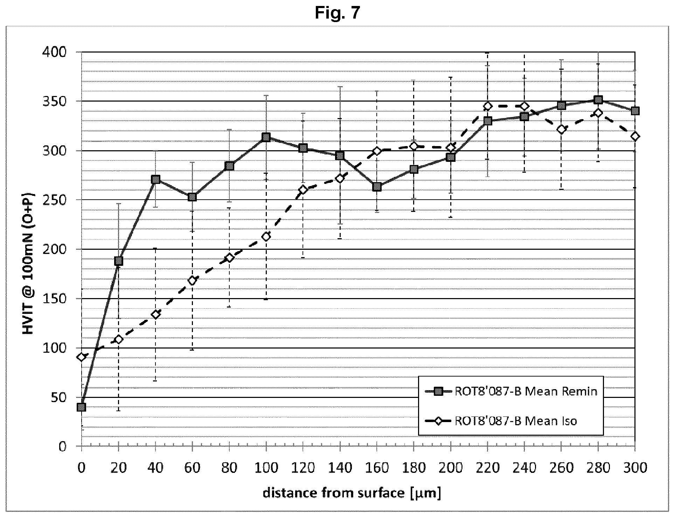

Fig. 7 zeigt die Härteprofile von Zähnen, die mit einer erfindungsgemäßen Zusammensetzung behandelt wurde (Beispiel 5; 0,1M Phenylalanin und 5% Ammoniumfluorid; durchgezogene Kurve), im Vergleich zu einer unbehandelten Schmelzprobe (gestrichelte Kurve). Die Härteprofile zeigen, dass die Behandlung mit der erfindungsgemäßen Lösung eine deutliche Härtezunahme vor allem der oberflächennahen Bereiche bewirkt.

### Ausführungsbeispiele

### Bestimmung des Remineralisationspotentials in kariogener Umgebung

Das Remineralisationspotential am Zahn wurde in einem Modell mit einer chemisch erzeugten Initialläsion in Rinderschmelz getestet. Dazu wurden Rinderzähne in Harz eingebettet, und mit SiC-Schleifpapier wurde unter Wasserkühlung der Schmelz freigelegt und poliert. Durch Lagerung für 14 bis 21 Tage bei 37°C in einer demineralisierenden Lösung wurde eine künstliche Läsion im Schmelz erzeugt. Die demineralisierende Lösung enthielt 50,0 mMol/l Essigsäure, 3,0 mMol/l KH₂PO₄, 3,0 mMol/l CaCl₂ · 2 H₂O, 1,0 ppm Methylendiphosphonsäure sowie als Konservierungsmittel 100 ppm Natriumazid. Der pH-Wert wurde mit KOH auf pH 5,0 eingestellt.

Die so erzeugten Läsionen wurden zur Hälfte mit Nagellack abgedeckt, und die freie Fläche wurde mit der erfindungsgemäßen Zusammensetzung behandelt. Anschließend wurde der Prüfkörper bei 37°C für weitere 7 Tage in der demineralisierenden Lösung bei pH 5,0 gelagert. Jede Behandlung wurde an je drei Zähnen durchgeführt.

Nach der Lagerung wurden die Prüfkörper kurz mit Wasser abgespült, trockengetupft und der Nagellack der isolierten Hälfte mit Ethanol entfernt. Von beiden Hälften wurde mit einem Nanoidenter die Oberflächenhärte gemessen. Hierzu wurde die Oberfläche der Prüfkörper mit Harz eingebettet, um anschließend mit einer Diamantsäge eine Scheibe des Zahnquerschnitts aussägen zu können. Nach dem Polieren des Querschnitts wurden von der behandelten und der isolierten Seite je drei Härteprofile vermessen (Eindrücke mit jeweils 20 µm Abstand vertikal bis in eine Tiefe von 300 µm, Berkovich-Identer, 100 mN Last, Beladung mit 400 oder 600 mN/Min., 2 s Haltezeit bei Fₘₐₓ). Mit den vom Gerät automatisch berechneten Vickershärtewerten (HV_{IT}) wurden Härteprofile erstellt.

### Beispiel 1

### Remineralisation mit Ammoniumfluoridlösung (Vergleichsbeispiel)

Remineralisation einer Initialläsion durch Applikation einer 5%igen Ammoniumfluoridlösung in Wasser (2,57 Gew% Fluorid; eingestellt auf pH 5,0) für 1 Minute. Nach dem Aufbringen der Lösung wurden die Zähne für 7 Tage in der demineralisierenden Lösung gelagert. Die Härteprofile dieser Probe sind in Fig. 3 wiedergegeben. Die gestrichelte Linie zeigt das Profil der mit Nagellack isolierten Hälfte der Schmelzproben. Die durchgehende Linie zeigt das Härteprofil der Schmelzprobe, die mit der Ammoniumfluoridlösung (ohne Aminosäurezusatz) behandelt wurde. Die Härteprofile in Fig. 3 zeigen in beiden Fällen eine Härteabnahme an der Oberfläche. Die Behandlung mit der Ammoniumfluoridlösung bewirkt zwar eine mäßige Remineralisation in einem engen oberflächennahen Bereich, in tieferen Bereichen wird aber eine weitere Demineralisation beobachtet.

### Beispiel 2

### Kontrollversuch mit einer unbehandelten Initialläsion (Negativkontrolle; Vergleichsbeispiel)

Die halbseitig mit Nagellack abgedeckten Zähne wurden für weitere 7 Tage in der demineralisierenden Lösung gelagert. Die nicht mit Nagellack bedeckte Seite wurde nicht behandelt. Die gestrichelte Linie zeigt das Profil der mit Nagellack isolierten Hälfte der Schmelzproben, die durchgehende Linie das Härteprofil der unbehandelten Schmelzproben. Die Härteprofile in Fig. 4 zeigen, dass die fortgesetzte Lagerung in der Demineralisierungslösung ohne Behandlung zu einer weiteren Abnahme der Härte führt. Dies bedeutet, dass die Läsion größer wird, also eine Demineralisation stattfindet.

### Beispiel 3

### Remineralisation mit einer lysinhaltigen Ammoniumfluoridlösung

Remineralisation einer Initialläsion durch Applikation einer 0,1M Lysinlösung mit 5% Ammoniumfluorid in Wasser (2,57 Gew% Fluorid; eingestellt auf pH 5,0) für 1 Minute. Nach dem Aufbringen der Lösung wurden die Zähne für 7 Tage in der demineralisierenden Lösung gelagert. Die Härteprofile in Fig. 5 zeigen, dass die Behandlung mit der erfindungsgemäßen Lösung eine deutliche Härtezunahme bewirkt (durchgehende Linie).

### Beispiel 4

### Remineralisation mit einer histidinhaltigen Ammoniumfluoridlösung

Remineralisation einer Initialläsion durch Applikation einer 0,1M Histidinlösung mit 5% Ammoniumfluorid in Wasser (2,57 Gew% Fluorid; eingestellt auf pH 5,0) für 1 Minute. Nach dem Aufbringen der Lösung wurden die Zähne für 7 Tage in der demineralisierenden Lösung gelagert. Die Härteprofile in Fig. 6 zeigen, dass die Behandlung mit der erfindungsgemäßen Lösung eine Härtezunahme in weiten Bereichen der Läsion bewirkt (durchgehende Linie).

### Beispiel 5

### Remineralisation mit einer phenylalaninhaltigen Ammoniumfluoridlösung

Remineralisation einer Initialläsion durch Applikation einer 0,1M Phenylalaninlösung mit 5% Ammoniumfluorid in Wasser (2,57 Gew% Fluorid; eingestellt auf pH 5,0) für 1 Minute. Nach dem Aufbringen der Lösung wurden die Zähne für 7 Tage in der demineralisierenden Lösung gelagert. Die Härteprofile in Fig. 7 zeigen, dass die Behandlung mit der erfindungsgemäßen Lösung eine deutliche Härtezunahme vor allem der oberflächennahen Bereiche bewirkt (durchgehende Linie).

## Patentansprüche

1. Zusammensetzung zur Anwendung in einem Verfahren zur Remineralisation von Zähnen, die
(a) einen flüssigen Träger,
(b) mindestens eine fluoridhaltige Komponente und
(c) mindestens eine Aminosäure enthält.

2. Zusammensetzung nach Anspruch 1, zur Anwendung in einem Verfahren zur therapeutischen Behandlung von Zähnen, insbesondere zur Remineralisation von Zähnen, zum Kariesschutz, zur therapeutischen Behandlung und Prävention initialer Kariesläsionen (Initialkaries), von weißen Flecken (white spots), von Zahnerosion, zur Härtung geschädigter Zähne und zur Wiederherstellung von Zahnhartgewebe.

3. Zusammensetzung nach Anspruch 1 oder 2, wobei die Zusammensetzung ein- bis dreimal, vorzugsweise einmal, auf den Zahn oder die Zähne aufgebracht und jeweils für einen Zeitraum von weniger als 5 Minuten, vorzugsweise 0,5 bis 2 Minuten, besonders bevorzugt 0,5 bis 1 Minuten und ganz besonders bevorzugt 1 Minute einwirken gelassen wird.

4. Zusammensetzung nach einem der vorhergehenden Ansprüche, die keine abrasiven und/oder keine polymeren Bestandteile enthält.

5. Zusammensetzung nach einem der vorhergehenden Ansprüche, die einen pH-Wert von 4 bis 7 aufweist, vorzugsweise 4 bis 6, und besonders bevorzugt auf pH 4,5 bis 5,5.

6. Zusammensetzung nach einem der vorhergehenden Ansprüche, wobei die fluoridhaltige Komponente Natriumfluorid, Kaliumfluorid, Ammoniumfluorid, Ammoniumbifluorid, Natriummonofluorophosphat, Kaliummonofluorophosphat, Tetra-n-butylammoniumdihydrogentrifluorid, Rubidiumfluorid, Cäsiumfluorid, Kaliumbifluorid (KHF₂), Silber-I-fluorid (AgF), Zinn-II-fluorid (SnF₂), Olaflur, Dectaflur oder eine Mischung davon ist.

7. Zusammensetzung nach einem der vorangehenden Ansprüche, die mehr als 1500 ppm, vorzugsweise mehr als 5000 ppm Fluorid (F⁻) enthält, bezogen auf die Gesamtmasse der Zusammensetzung.

8. Zusammensetzung nach einem der vorhergehenden Ansprüche, wobei die Aminosäure Lysin, Histidin, Phenylalanin, Arginin, Glutamin, Asparagin, Tyrosin, Glycin, Serin, Cystein, Threonin, Alanin, Valin, Methionin, Leucin, Isoleucin, Prolin, Tryptophan, Glutaminsäure, Asparaginsäure, Hydroxyprolin oder eine Mischung davon ist.

9. Zusammensetzung nach einem der vorangehenden Ansprüche, die 0,01 bis 0,5 mol/l, vorzugsweise 0,025 - 0,2 mol/l Aminosäure enthält, bezogen auf das Gesamtvolumen der Zusammensetzung.

10. Zusammensetzung nach einem der vorhergehenden Ansprüche, die als fluoridhaltige Komponente (b) Ammoniumbifluorid, Tetra-n-butylammoniumdihydrogentrifluorid, Olaflur (INN), Dectaflur (INN), Ammoniumfluorid oder eine Mischung davon und/oder als Aminosäure (c) Histidin, vorzugsweise Phenylalanin oder Lysin oder eine Mischung davon enthält.

11. Zusammensetzung nach einem der vorhergehenden Ansprüche, die als flüssigen Träger Wasser, Ethanol, Isopropanol, Aceton, Methanol, Propylenglycol oder eine Mischung davon, vorzugsweise Wasser enthält.

12. Zusammensetzungen nach einem der vorhergehenden Ansprüche, die
(a) 50 bis 99,5 Gew.-%, vorzugsweise 75 bis 99 Gew.-%, besonders bevorzugt 85 bis 98 Gew.-% und ganz besonders bevorzugt 86 bis 96 Gew.-% flüssigen Träger,
(b) 0,15 Gew.-% bis 20 Gew.-%, vorzugsweise 0,5 Gew.-% bis 10 Gew.-% und besonders bevorzugt 1 bis 5 Gew.-% (bezogen auf F⁻) mindestens einer fluoridhaltigen Komponente, jeweils bezogen auf das Gesamtgewicht der Zusammensetzung,
(c) 0,01 bis 0,5 mol/l, vorzugsweise 0,025 bis 0,2 mol/l und besonders bevorzugt 0,05 bis 0,1 mol/l mindestens einer Aminosäure, bezogen auf das Gesamtvolumen der Zusammensetzung, enthält.

13. Zusammensetzung nach Anspruch 12, die zusätzlich
(d) ggf. Mittel zur Einstellung des pH-Werts und/oder
(e) 0,01 bis 5 Gew.-%, vorzugsweise 0,1 bis 3 Gew.-% und besonders bevorzugt 0,5 bis 2 Gew.-%, bezogen auf das Gesamtgewicht der Zusammensetzung, weitere Additive enthält.

14. Zusammensetzung nach einem der vorhergehenden Ansprüche, wobei das Verfahren die folgenden Schritte umfasst:
(i) Optionale Vorbehandlung des Zahns oder der Zähne,
(ii) optionales Anätzen des Zahns oder der Zähne und
(iii) Applikation einer erfindungsgemäßen Zusammensetzung.

15. Verwendung einer Zusammensetzung gemäß einem der Ansprüche 1 bis 12, zur Remineralisation von Zähnen, zum Kariesschutz, zur Behandlung und Prävention initialer Kariesläsionen (Initialkaries), von weißen Flecken (white spots), zur Verhinderung oder Behandlung von Zahnerosion, zur Härtung geschädigter Zähne und zur Wiederherstellung von Zahnhartgewebe.
